# EUROPEAN PATENT APPLICATION

(11) **EP 4 660 940 A1**
(43) Date of publication of application: **10.12.2025**
(21) Application number: 25177280.2
(22) Date of filing: 19.05.2025
(51) Int. Cl.: G06T 7/00

(54) **CLASSIFICATION OF SUBSTRATE REGIONS**

(30) Priority: 29.05.2024 US 202418677692
(71) Applicant: FEI COMPANY, Hillsboro, OR 97124 (US)
(72) Inventor: Mutch, Joshua, Salem (US); Bahm, Alan, Hillsboro (US); Balashov, Konstantin, Beaverton (US); Gilbert, Joshua, Dublin (US); Hoshyargar, Faegheh, Oregon (US)
(74) Representative: Boult Wade Tennant LLP

(57) **Abstract**

Methods and apparatus are disclosed for classifying regions of a substrate prior to performing an analytic procedure involving analyte particles supported on the substrate. Regions of the substrate are sparsely scanned using low-energy electron point projection (LEEPP) imaging. Regions are classified as suitable for the analytic procedure (or not) based on defects visible in the respective images. Given one suitable region, neighboring regions are scanned to increase the yield of suitable regions. Based on a distribution of suitable regions, a deposition pattern is planned, and analyte is deposited according to the plan. Following deposition, suitable regions are scanned again to identify or count visible analyte molecules visible. Based on numbers of analyte molecules found, regions are earmarked for analyte characterization, e.g. by low-energy electron holography and reconstruction. A trained machine learning classifier provides consistent, accurate image classification across a range of defect types.

## Description

### BACKGROUND

Graphene is emerging as a suitable substrate on which proteins can be supported for low-energy electron holography (LEEH) and other similar transmission mode analytic procedures. However, surface contaminants and structural defects can compromise imaging performance and can reduce yields. Accordingly, there remains a need for improved technologies to improve yields in protein structure studies and similar applications.

### SUMMARY

In brief, examples of the disclosed technologies use low-energy electron point projection (LEEPP) imaging to qualify regions of a substrate as suitable for analyte deposition and characterization. In one aspect, a sparse scan of regions can balance coverage of a substrate area with scan time, and a secondary scan in the vicinity of a suitable region can efficiently increase the number of suitable regions available for analyte studies. In another aspect, an analyte deposition pattern can be established based on a distribution of the suitable regions. In a third aspect, classification prior to analyte deposition can be used to identify regions suitable for analyte deposition, while classification after analyte deposition can be used to identify regions having suitable amounts of analytes for further studies. In a fourth aspect, images of scanned regions can be classified by a trained machine learning tool.

The foregoing and other objects, features, and advantages of the invention will become more apparent from the following detailed description, which proceeds with reference to the accompanying figures.

### BRIEF DESCRIPTION OF THE DRAWINGS

FIG. 1 is a diagram of an example environment in which the disclosed technologies can be practiced.
FIG. 2 is a flowchart of an example analysis workflow according to the disclosed technologies.
FIG. 3 is a diagram illustrating an example of LEEPP imaging according to the disclosed technologies.
FIG. 4 is a set of images illustrating examples of various classes of LEEPP images of a graphene substrate.
FIG. 5 is a pair of schematic drawings illustrating an example application of the disclosed technologies.
FIG. 6 is a set of example images according to the workflow of FIG. 2.
FIG. 7 is a diagram of a substrate illustrating example regions and example deposition patterns according to the disclosed technologies.
FIG. 8 is a flowchart of a first example method according to the disclosed technologies.
FIG. 9 is a flowchart of a second example method according to the disclosed technologies.
FIG. 10 is a flowchart of a third example method according to the disclosed technologies.
FIGS. 11A-11B are dataflow diagrams for respective phases of the method of FIG. 10.
FIG. 12 is a flowchart of an example extension to the method of FIG. 10 according to the disclosed technologies.
FIG. 13 illustrates a generalized example of a suitable computing environment in which embodiments, techniques, and technologies pertaining to substrate characterization or analysis workflow can be implemented according to the disclosed technologies.

### DETAILED DESCRIPTION

### Introduction

Graphene is emerging as an attractive electrically conductive substrate material, in particular for protein structure studies. Graphene monolayer and bilayers can be suspended over apertures in a support member, and can have electron transparency greater than 70% or 30%, respectively, at typical low electron energies (50 - 600 eV) used in point projection imaging. That is, a high proportion of electrons can pass through a thin graphene layer without interaction. At the same time, electrical conductivity of 1-2 × 10⁶ S/m is sufficiently high to prevent charge build-up and to provide a planar ground plane for field shaping between an electron emitter and the substrate.

Analyte particles, such as proteins, can be deposited on the graphene and studied by holography and other techniques. Unlike cryogenic transverse electron microscopy (cryoTEM), where analyte particles are immobilized, studies according to the disclosed technologies can be performed without analyte immobilization, enabling dynamic particle behavior to be studied.

However, analyte studies require graphene to be clean, e.g. free of defects. A defect can interfere with measurements of a nearby analyte particle. Some defects also pose a risk of arcing under high-magnification conditions employing high electric fields. Arc damage can result in prohibitive equipment downtime of hours or even days, in addition to wasting a substrate and any deposited analyte.

Manufacturing processes often deliver graphene that is not clean enough for analyte studies. For example, graphene foils can be produced by deposition on a metal substrate, and can be transferred to a target substrate in a four-step process: (1) polymethyl methacrylate (PMMA) application onto the graphene, (2) etch removal of the original metal substrate, (3) transfer to the target substrate, and (4) etch removal of the PMMA backing. This transfer process can leave PMMA or solvent residues, or other contaminants, on the transferred graphene. Repeated handling of the graphene, even with such techniques as an ultra-high vacuum (UHV) briefcase, introduces additional opportunities for contamination.

Contaminants are termed "extrinsic defects" on the graphene. Graphene can also suffer from "intrinsic defects" which are defects in the graphene crystal structure, and can manifest as ruptures, cracks, strands, bubbles, crystal dislocations, or other structural defects. Substrates of other 2D materials can also be feasible, but face similar issues.

Some examples of the disclosed technologies take an approach of characterizing substrate regions for cleanliness. Regions which are free of defects, or which have defects below a severity threshold, can be earmarked as suitable for analyte deposition. After deposition, these regions can be imaged again. Any new particles observed can be deemed analyte particles with a high degree of confidence.

Notably, entire analyte characterization workflows described herein can be performed in situ in a single process chamber, including substrate characterization, analyte deposition, analyte mapping, and analyte characterization. Substrate handling can be minimized, and ultra-high vacuum below about 1 micropascal (7.5 nanotorr) can be maintained throughout.

While cleaning technologies exist, they can have limited success. Sometimes, only 5-10% of a graphene substrate may be clean enough for analyte characterization. However, if analyte particles can be deposited in locally clean regions of a substrate, then a requirement to have an entire substrate clean can be avoided. Accordingly, some disclosed technologies take an approach of scanning regions of the substrate to identify regions that are locally clean.

Low-energy electron point projection (LEEPP) is an attractive technique for imaging a graphene substrate, because good contrast can be obtained between transparency of the graphene and feature visibility of both intrinsic and extrinsic defects. Moreover, image quality and magnification can be sufficient for characterizing defects. An alternative technique, atomic force microscopy, is orders of magnitude slower, taking 10-20 minutes to scan a tile which can be imaged by LEEPP in about 5 seconds, including positioning.

Deposition apparatus can deposit over a fairly broad pattern having transverse extent 50-500 µm, often 100-200 µm, in part because of positioning drift between an analyte source and a substrate. Accordingly, it is desirable that the regions scanned cover the transverse extent of the substrate. As described further herein, there is a tradeoff between high magnification (for better defect visibility) and large field of view (for efficient coverage of a substrate with fewer images and hence a shorter time duration). Regions of a few µm transverse extent (e.g. 3-10 µm or about 4-6 µm) are practical for defect visibility, but scanning an entire substrate of a few hundred µm transverse extent can take a number of hours. In varying scenarios, a comprehensive scan of such a substrate can take 4 - 72 hours, or 12 - 24 hours.

Some examples of the disclosed technologies take an approach of sparse scans to reduce scan time while covering the transverse extent of the substrate. The substrate area can be divided into tiles. Sparse sampling of tiles can vary from 1-in-4 to 1-in-400 of the available tiles, and the total time to scan a substrate can be reduced by a factor of 4 to 400. At about 1-10 seconds per tile, an entire sparse scan can be completed in under an hour, or even just a few minutes. In varying scenarios, a sparse scan can take 40 s - 6 hours, 2 minutes - 2 hours, or 10 - 40 minutes.

Often, defects are prevalent, and the fraction of regions found to be defect-free can be in a range 1-20%, sometimes about 5%. Further, the density of defect-free regions can vary considerably over the substrate.

To increase yield of analyte characterization data, it may be desirable to identify more clean regions than can be found with the sparse scan. Defects can be spatially correlated, meaning that a clean tile is more likely to have additional clean neighboring tiles than a defect-laden tile. Accordingly, some examples of the disclosed technologies take an approach of performing a secondary scan around an identified clean tile, to increase the total count of clean tiles which can be utilized for subsequent analyte studies.

With sparse and secondary scans complete, a number of tiles may be identified as suitable for an analytic procedure (e.g. deposition and characterization of an analyte such as a protein). Some examples of the disclosed technologies take an approach of planning a deposition pattern to optimize utilization of the suitable tiles. As described further herein, various factors can be considered for optimizing the deposition pattern. Because a deposition pattern can be nonuniform (often bell-shaped), the deposition pattern can be arranged to optimize suitable tiles that are within a preferred zone of the deposition pattern. Particularly, near the edges of the deposition pattern, a tile may receive too few (or even zero) analyte particles, which can be sub-optimal. Conversely, near the center of the deposition pattern, a tile may receive too many analyte particles, whose signals can interfere with each other during subsequent characterization. Parameters of the deposition beam and relative positioning of source and substrate can be configured to obtain a suitable deposition pattern.

Then, analyte deposition can be performed according to plan, resulting in a number of suitable tiles having analyte particles ready for characterization. However, it can be helpful to map these tiles, so as to avoid wasting characterization time on tiles that have no analyte particles or that have too many analyte particles.

Some examples of the disclosed technologies take an approach of repeating an imaging scan (e.g. LEEPP) over just the suitable tiles, to identify a subset of the suitable tiles actually having one or more visible analyte particles suitable for characterization.

Then, analyte particles in this subset of tiles can be characterized. Low-energy electron holography can be used, using same or similar equipment as for the LEEPP imaging, but configured for higher magnification. The holograms can be processed by computer to reconstruct a structural description of the analyte particle.

In another aspect, consistent classification of LEEPP images can pose a challenge. When performed by humans, two persons can disagree, or even a single person can change their thresholds for identifying defects over time. Machine vision tools employing e.g. edge detection or shape detection techniques can be efficient but, because they can be based on heuristics, can fail when they encounter images outside a limited range. LEEPP imaging of 2D materials is also prone to variations in illumination across a single image or between images, which can pose additional difficulty to machine vision heuristics.

Accordingly, some examples of the disclosed technologies take an approach of using a trained machine learning tool to classify LEEPP images of substrate regions. The trained ML tool can be implemented as a convolutional neural network (CNN) trained on a labeled dataset having LEEPP images of a similar substrate, classified by a human expert. This approach can provide consistent classification, can learn to mimic classification by a human expert, and can provide more accurate results (in terms of agreement with the human expert) than machine vision heuristics. Additionally, the ML workflow can be easily adapted to new substrates, image sets, or target classifications as technologies and the needs of analyte studies evolve.

In tests, a trained ML classifier described herein has demonstrated accuracy over 96%, with the discrepancies predominantly occurring in images for which the human expert's classification also had low confidence. For images having high confidence in the human expert's classification, the ML classifier's accuracy was well over 99%.

The description above is illustrative and does not attempt to capture all innovative features disclosed herein. Additional details are described below in context of the Figures. Moreover, any example of the disclosed technologies can adopt, omit, or vary any of the features described above, in any combination. Particularly, disclosed technologies can be applied to other substrate materials or other imaging modalities.

### Terminology

The usage and meaning of all quoted terms in this section applies throughout this disclosure unless clearly indicated otherwise or repugnant to the context. The terminology below extends to related word forms.

An "analyte" is a material species used as a sample for an analytic procedure. In some disclosed examples, the analytic procedure can be holographic imaging of an analyte using an electron point projection microscope. Non-limiting examples of analytes can include macromolecules such as proteins, lipids, nanotubes, nucleic acids, polymers, capsids, biomolecule-ligand complexes, protein-protein complexes, RNA, viruses, viral-like assemblies; molecular components; bioinspired materials; or inorganic nanoparticles. Other analytic procedures can include electron backscatter analysis, electron microscopy, etching, imaging, mass spectrometry, material analysis, metrology, nanoprobing, spectroscopy, sample preparation, or surface preparation. Equipment used to perform analytic procedures is termed an "analytic instrument." Analyte particles are often molecules, but this is not a requirement and, in some examples, analyte particles can be clusters or fragments of molecules.

An "aperture" or "pore" is a hole in a solid object providing a clear straight line path through the object, from one external surface of the object to an opposite external surface. A material having multiple apertures is dubbed "porous." In some examples of the disclosed technologies, a substrate can be supported on a porous support such as holey silicon nitride ("holey SiN") membrane having aperture diameters commonly in a range 400-1000 nm.

A "beam" is a directional flow of particles or energy. Common beams of interest in this disclosure are electron beams, ion beams, or light beams, but the term is not limited thereto. Ion beams and electron beams are flows of ions or electrons respectively; each particle of such a beam carries electric charge and is a "charged particle." The "energy" of a charged particle beam is the mean kinetic energy of the individual charged particles in the beam. A light beam is a flow of electromagnetic energy, which can be regarded as waves or as (particulate) photons. A beam can have finite extent transverse to its principal longitudinal direction of flow. A line joining the centroids of two or more transverse cross-sections of a beam is an "axis" of the beam, or "beam axis." An ion beam which deposits material on a substrate is a "deposition beam," and its axis is termed a "deposition axis."

"Charged particle optics" refers to devices which can change the propagation characteristics of a charged particle beam. Exemplary propagation characteristics can include direction of propagation, focusing (e.g. convergence or divergence), cross-sectional profile, energy, or dispersion. Such devices can apply electric or magnetic fields to the beam and are collectively termed "electromagnetic devices." An "electromagnetic deflection element" is an electromagnetic device configured to control direction of propagation of a charged particle beam.

"Classify" and "classification" refer to an act of assigning an item to one or more of a finite predetermined set of choices. In some examples of interest herein, the classified item can be an imaged region of a sample, and each of the choices can indicate a type or severity of defect(s) in the region. In varying examples, classification can be performed by trained machine learning software, by other automated software, or by a user (e.g. using interactive software). A software program performing classification is termed a "classifier". A class assigned to training data is dubbed a "label."

"Cleanliness" is a parameter characterizing severity of defects of a substrate or region thereof. Cleanliness can have a numerical value, which can vary directly or inversely as the amount of defects, or can have a logical value (e.g. clean or not clean).

A "contaminant" is an undesirable material species on or in a sample. In some disclosed examples, contaminants on a graphene sample can include organic residues from a poly-methyl methacrylate (PMMA) transfer procedure. A contaminant can adversely affect a downstream analytic process, e.g. by decreasing resolution or otherwise degrading an image performed using the sample. Contaminants can also cause equipment damage through arcing.

A "controller" is an electronic device coupled to one or more actuators to effect a change in a physical parameter, or coupled to one or more sensors to monitor a physical parameter. Some controllers can include a microprocessor which can be programmed to execute machine readable instructions. The descriptions herein of computing devices are generally applicable to such controllers. Such controllers can include additional electronic circuitry such as analog-to-digital converters (ADCs), digital-to-analog converters (DACs), switches, comparators, filters, or amplifiers. Other controllers can include analog circuitry without any microprocessor.

A "criterion" is a condition or basis for making a categorical determination.

In context of a substrate region, a "defect" refers to any characteristic of the substrate region that adversely effects its utility for supporting, imaging, or performing other analysis on an analyte. A defect is "visible" if it is discernible on a LEEPP image of the substrate region. Particularly, a defect over an aperture in a holey SiN support may be visible, yet a similar defect away from any aperture may not be visible because the underlying Si support is opaque to a low energy electron beam. An "intrinsic" defect is part of the substrate (graphene) structure, such as a bubble, rupture, crack, dislocation, ridge, or void. An "extrinsic" defect is on a surface of the substrate, and can be a contaminant in the form of a particle, adsorbate, droplet, or film. A previously deposited analyte molecule can also be an extrinsic defect with respect to another, future, analyte deposition.

"Deposition" refers to an act of adhering material (e.g. a protein or other analyte) onto a substrate. The spatial distribution of the deposited material is termed a "deposition pattern."

A "detector" is an apparatus for measuring a received signal, which can be in the form of light, an electrical signal, or an electron beam. Detectors can incorporate photodiodes, complementary metal oxide semiconductor (CMOS) elements, charge coupled devices (CCD), microchannel plates, photomultipliers, or similar devices, singly or in arrays. Some detectors can be pixelated, and can form an image from a spatially distributed signal. However, a pixelated detector is not a requirement for forming an image. In other examples, the spatial variation of an image can be obtained e.g. by scanning a narrow or point beam across a sample (as in an SEM), or by moving a small detector across a spatially distributed signal. A detector can be part of an analytic instrument such as a spectrometer or an electron point projection microscope.

An "electron microscope" is a type of analytic equipment in which a sample is illuminated by an electron beam, and resulting particles or electromagnetic radiation are used to form a spatially resolved image. An "electron point projection microscope" images a sample (e.g. an analyte) using a divergent electron beam and detecting transmission through or around a sample, or diffraction or scattering from the edges of the sample. A scanning electron microscope (SEM) images a sample surface based on reflected, secondary, or backscattered particles or radiation from one or more surfaces of the sample. An electron microscope can include an electron source (sometimes, "electron emitter") and an imaging detector.

A "hologram" is an image incorporating amplitude and phase information of interfering waves. The waves can be electromagnetic waves (e.g. light waves) or de Broglie waves of interfering electrons (e.g. in an electron point projection microscope). A variety of techniques exist to reconstruct a spatial description of the imaged object. As an example, the hologram can be Fourier transformed into a momentum space, propagated in the momentum space, then inverse transformed back to physical space to recover a spatial description of the imaged object.

An "image" is a two-dimensional representation of a parameter value over a region of interest of a sample. In examples, the region of interest can be a tile of a substrate, a portion of the substrate over an aperture in a support, or a smaller region enclosing one or a few analyte molecules. The parameter can be an amplitude of electrons reaching a detector. An "imager" is an apparatus for generating an image, and can include an illumination source and a detector.

"Low energy electron point projection" ("LEEPP") is a technique for imaging a sample using an electron beam whose energy is in a range 50 - 1000 eV. Voltage and magnification can be independently varied as described herein, although lower magnification imaging (wider field of view) can be performed with higher voltages. Thus, up to about 600 eV is suitable for tile of 1-10 µm transverse extent, and LEEPP imaging of an entire 500 µm substrate can be performed with up to about 1000 eV energy. At higher energies, defects can become transparent and the contrast between clean graphene and defects can lessen. At higher magnification, interference effects in the image can become more prominent, and the image can be regarded as a hologram. In this regime, LEEPP can be termed "low energy electron holography" ("LEEH").

"Machine learning" (or "ML") denotes a technique for improving performance of a software tool through experience (dubbed "training"), and that tool is dubbed a "machine learning tool." The qualifier "trained," as an adjective, indicates that an ML tool has undergone training to attain performance at least equal to a predetermined threshold. Training can be performed in a "training phase," in which training data (for which desired outputs are known) is applied at the input to the tool, and deviations between the tool output and the desired output are used to adjust parameter values within the ML tool, e.g. by backpropagation. After being trained, the ML tool can be fed fresh data and its outputs can be used as needed. This phase is sometimes termed an "inference phase." A neural network is an example of a software tool that can be trained by machine learning.

A "major surface" of a substrate or sample is a surface of the substrate or sample whose area is not substantially exceeded by any other surface of the substrate or sample. For convenience of description, substrates or samples are considered to have top and bottom major surfaces, with the bottom surface affixed to a support. In some examples, a low energy electron beam can be directed to a top surface of a substrate and analyte deposition can be performed on the bottom surface, e.g. through apertures in the support.

A "molecule" is the smallest unit of a material having the same composition and chemical properties as the material. While many molecules contain two or more atoms (CO2, O2), this is not a requirement and a single atom (e.g. He) can also be regarded as a molecule. Some molecules described herein can be in an ionized state, e.g. in a deposition beam.

First and second regions are "neighbors" if they are adjacent to one another, including along a diagonal. A gap between two regions does not preclude their being neighbors, so long as no other region is present in that gap.

A "neural network" is an artificial network of "units" (or "cells") that has linkages modeled on behavior of biological neurons and that can be implemented by a software program on a computer. A neural network is an example of a machine learning tool. Some neural networks described herein can be "convolutional neural networks" (or "CNN"s) which have couplings between cells independent of the spacing between the cells. A CNN is a multi-layer neural network incorporating at least one convolutional layer, the connectivity and parameters of which apply a convolution operation uniformly across cells of a preceding layer to obtain the instant layer.

A "notification" is a message for which a substantive response may or may not be required. A notification can be presented as a computer communication (e.g. over a bus or network, or written in a shared memory location), as a visual display on a graphical user interface (GUI) or annunciator, or by audio or haptic means. In contrast, a "request" is a message for which a substantive response (e.g. beyond simple acknowledgement) is expected.

A "parameter" is an attribute or property of a physical system which can be measured or observed. A result of such measurement or observation is a "value" of the parameter. A parameter can also be an attribute or property of the physical system which can be controlled. In this context, a setting applied to the parameter is its value. A parameter can have one or more "values". While parameters often have numerical values, this is not a requirement, and some parameter values can be logical values (e.g. whether the parameter is above or below a threshold, or On or Off), categorical variables (e.g. classification of defect type or severity), strings (e.g. describing the parameter), or data structures (e.g. a LEEPP image).

A "protein" is a molecule containing a chain of amino acids linked by peptide bonds. For protein analytes, an analyte particle is a protein molecule.

A "region" is a contiguous generally two-dimensional extent of a substrate, and can include both top and bottom surfaces, the volume enclosed therein, and extrinsic defects on either surface. In various examples, a region can be a portion of the substrate suspended over a single aperture, or a tile covering multiple apertures. Descriptions of regions herein encompasses both apertures and larger tiles, as well as regions which are smaller than a single aperture. A "suitable" region is one which meets a defect severity criterion (e.g. with minor or no defects) and can therefore be used for subsequent analysis operations, including analyte deposition.

A "region of interest" (ROI) is a portion of a substrate or sample that includes a feature or structure (e.g. an analyte molecule, a substrate defect, or a defect-free substrate region) that is the subject of imaging or other analysis. The term ROI does not refer to any human interest.

"Rotation" refers to a change in angular orientation of one object relative to another object or relative to a fixed coordinate system. In the former case, rotation can be accomplished by moving either one or both of the objects. In some examples, a deposition axis of an analyte ion beam can be rotated relative to a substrate. Such a rotation can have two degrees of freedom: a tilt relative to a surface normal of the substrate, and an azimuthal rotation about the surface normal.

A "sample" is a physical object upon which an analytic, manufacturing, or preparatory procedure is performed. A "substrate" is another physical object, distinct from the sample, upon which the sample is mounted for such procedure. Analytic, manufacturing, or preparatory procedures can also be performed on the substrate. In a sequence of operations, a sample on which one operation is performed can be a substrate for another operation. To illustrate, the disclosed technologies can be used to characterize a graphene sample mounted on a holey silicon nitride substrate. The disclosed technologies can also be used to prepare an analyte sample on a characterized region of a graphene substrate. Thereafter, holographic analysis can be performed on the analyte deposited onto the graphene, the latter now in the role of a substrate. In examples, a sample and substrate can be successively affixed to a support (e.g. holey SiN), a mount (e.g. a clamping fixture), and a stage. Some graphene substrates described herein can have transverse extent about 200-1000 µm, or about 400-500 µm.

"Severity" is a measure of the extent to which one or more defects impact useability of a substrate region, e.g. for an analytic procedure. In varying examples, severity can be expressed as a binary variable, a categorical variable, or as a number.

"Software" refers to computer-executable programs, instructions, or associated data structures. Software can be in active or quiescent states. In an active state, software can be loaded into memory, or can be undergoing execution by one or more processors. In a quiescent state, software can be stored on computer-readable media, awaiting transmission or execution.

A "source" is an apparatus configured to deliver material or energy. Exemplary sources describe herein include electron sources, analyte sources, or light sources. To illustrate, imaging can be performed using an electron beam generated by an electron source. An exemplary analyte source can deliver a beam of ionized proteins onto a clean graphene sheet for subsequent imaging. A light source can generate light of one or more wavelengths.

"Sparse" refers to a sample whose cardinality is at most 25% the cardinality of the population or space of which it is a part, and whose members form at least two disjoint groups. In some examples, a sparse set of images has a transverse separation distance (e.g. gap) between images greater than or equal to a transverse extent of each image.

A "stage" is an apparatus to which a substrate or sample can be attached and having actuators for adjusting a position or orientation of the sample. A "multi-axis stage" has multiple degrees of freedom, offering spatial adjustment of a sample in varying combinations of translation or rotation.

"Steer" refers to an act of changing direction of a beam relative to a physical object or relative to a fixed reference frame. Steering of a charged particle beam can be accomplished using electromagnetic deflection elements.

"Training" refers to a process of determining values (coefficients) to be applied within an ML tool, e.g. at neurons of a neural network, so as to render the ML tool operable for its desired functionality. In the context of a classifier, training can be performed using a training data set comprising images for which a desired classification is already known. Comparison of actual output of a trainee ML classifier with the desired classification can provide a loss function, which can be propagated backward through the ML classifier, applying gradient descent or another established technique to tune the coefficients at each layer. As training proceeds, the classifier outputs can converge to the desired classifications, and the magnitude of the loss function can decrease. When the loss function has decreased to below a predetermined threshold, the classifier can be validated and deemed "trained." The trained classifier can be deployed to classify new data (e.g. LEEPP images) for which a correct classification is not already known.

The term "translation" refers to a change in position of a physical object relative to another object or relative to a fixed coordinate system. In the former case, translation can be accomplished by moving either one or both of the objects. Although a translation from coordinates (x1, y1, z1) to (x2, y2, z2) can be performed in a straight line (i.e., as a "linear translation"), there is no requirement to do so unless expressly stated. A translation can be performed in a circular, sinuous, or other path. Similarly, a translation neither requires nor precludes any change in orientation. However, a change in orientation without a change in position is not considered a translation.

The terms "top," "bottom," "up," "down," "above," "below," "horizontal," "vertical," and the like are used for convenience, with respect to a common configuration in which an exposed major surface of a horizontal sample has an outward normal directed upward, e.g. the sample is accessible to process tools from above. "Transverse" refers to directions within or parallel to a major surface of the sample. "Longitudinal" refers to a direction along a beam axis. One of ordinary skill will understand from this disclosure that a choice of actual orientation can be varied without departing from the scope of the disclosed technologies.

A "two-dimensional material" (or, "2D material") is a thin sheet of material having a thickness one to ten atomic layers. Some 2D materials have high transparency to electrons, light, or other bands of electromagnetic radiation. Some 2D materials have a single atomic layer and are termed "single-layer" or "monolayer." Some 2D materials have two atomic layers and are termed "double-layer" or "bilayer." Examples of the disclosed technologies use single layer graphene as a substrate. Bilayer graphene or other 2D materials can also be used. Exemplary alternatives to graphene can include hexagonal boron nitride, molybdenum disulfide, other transition metal dichalcogenides, phosphorene, silicene, platinum, nickel, melamine, 2DPA-1, or similar sheet polymers.

"Validation" is a process or act of verifying that a software module meets its specification. In the context of an ML classifier, a specification can require that agreement, between the classifier output and a human expert, meet or exceed a predetermined threshold. The threshold can be in a range 80-100%, e.g. 90%, 95%, 98%, 99%, 99.5%, 99.8%, or 99.9%.

### Example Environment and Analysis Workflow

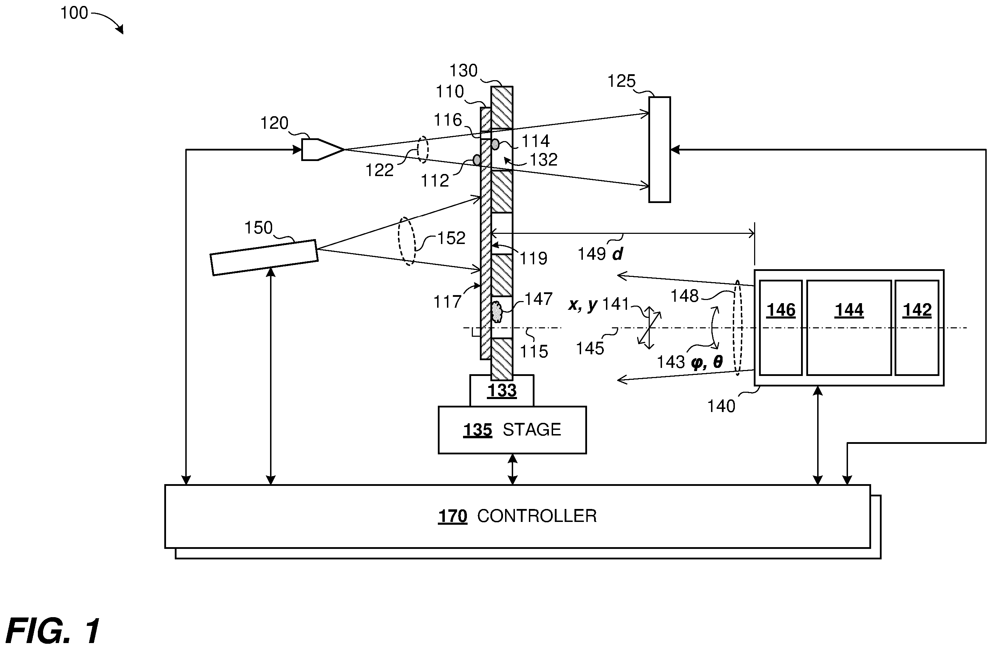

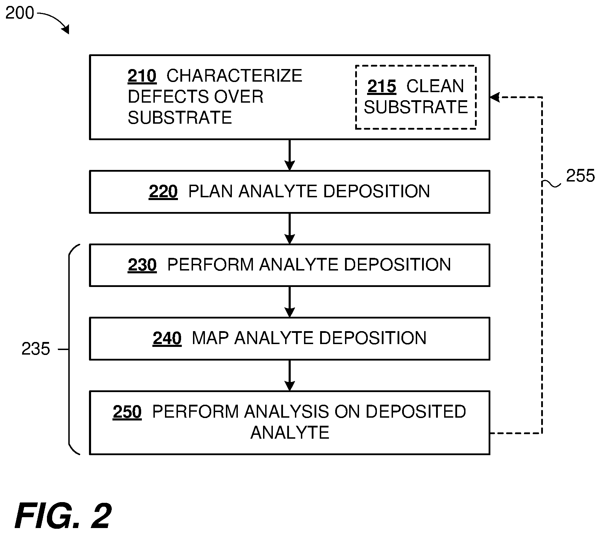

FIG. 1 is a diagram 100 of an example environment in which the disclosed technologies can be practiced. Shown in FIG. 1 are a substrate with exemplary defects, mounting devices, analytic equipment, and controllers. FIG. 1 is described in conjunction with FIG. 2, which is an example of an analysis workflow on an analyte species.

Substrate 110 can be affixed to support 130, which in turn can be held by mounting bracket 133 on stage 135. Stage 135 can be operated by controller 170 to provide rotation or translation of substrate 110 with one or more degrees of freedom. Substrate 110 can incorporate graphene or another 2D material. Support 130 can incorporate silicon (e.g. in the form of holey silicon), silicon nitride (SiN), or another porous material. As illustrated, apertures 132 are regularly distributed over support 130, but this is not a requirement.

Substrate 110 can have an assortment of defects. Exemplary defects can include extrinsic defects such as contaminant particles 112, 114 on top or bottom surfaces of substrate 110 respectively, or intrinsic defect 116, shown as a rupture. The illustrated defect positions are exemplary. Other types of intrinsic defects can be located on a surface of substrate 110, and extrinsic defects can sometimes penetrate through an aperture of substrate 110. Defects are described further herein.

Also shown in FIG. 1 is a LEEPP imaging subsystem, comprising electron emitter 120, configured to produce low-energy electron beam 122, and electron detector 125, which can be a pixelated detector. Controller 170 can control emitter 120 and can read out detector 125. The LEEPP imaging subsystem can be used to characterize regions of substrate 110 according to defect severity, e.g. at block 210 of FIG. 2. Low-energy electrons 122 can penetrate through 110, yet can render defects 112, 114, 116 visible. Representative LEEPP images showing the presence of such defects are described further herein. FIG. 1 shows beam 122 incident on top surface 117 of substrate 110, but this is not a requirement and, in other examples, LEEPP imaging can be performed from the opposite side of substrate 110.

For clarity of illustration, beam 122 is shown illuminating a substrate region corresponding to a single aperture 132 in support 130, but this is not a requirement. In examples, a suitable LEEPP imaging geometry can be constrained by several factors. In one aspect, larger beam widths can suffer from variations in beam intensity across a transverse cross-section, with attendant degradation in image quality. A practical transverse size of detector 125 can be another constraint. For a given practical detector size and beam width, there can be a tradeoff between magnification and imaging area. Thus, if substrate 110 is moved closer to source 120, keeping detector 125 fixed, magnification increases but the field of view (e.g. the imaged region of substrate 110) becomes smaller, thereby requiring more time to scan a given amount of substrate surface area and reducing the scanning efficiency. Conversely, if substrate 110 is moved closer to detector 125, keeping emitter 120 fixed, the field of view increases but magnification decreases. At low magnification, significant defects may not be discernible, or may be prone to incorrect classification.

Substrate 110 can desirably be used as a support for analyte particles 147. To this end, analyte deposition source 140 is also shown. Illustratively, source 140 can incorporate analyte source 142, conditioner 144, and delivery subsystem 146, some or all of which can be controlled by controller 170. In some examples, source 142 can be an electro-spray ionizer or a MALDI (matrix assisted laser desorption ionization) source, which can be configured to introduce analyte ions into conditioner 144. Conditioner 144 can be a mass spectrometer, which can act as a mass filter, forwarding only particles within a narrow window about a desired m/Z ratio, to subsystem 146. For ionized analytes, delivery subsystem 146 can be implemented with charged particle optics. In examples, optics 146 can deliver a collimated beam 148 having 100-500 µm beamwidth through a series of electromagnetic lenses.

In examples, electric fields can be applied to analyte stream 148 to apply braking as analyte ions approach substrate 110, and provide a soft landing with particle kinetic energy about 5-20 eV, or less than about 50 eV, at a surface potential about 0.2-5 V, commonly about 1 V. Soft landing can prevent fragmentation of analyte molecule 147 upon impact with substrate 110, and can also prevent damage to substrate 110 from this impact. FIG. 1 shows beam 148 incident on bottom surface 119 of substrate 110, but this is not a requirement and, in other examples, analyte deposition can be performed from the opposite side of substrate 110.

Also shown in FIG. 1 are certain attributes of analyte deposition beam 148, which is shown having a deposition axis 145. Notably, beam 148 can be translated relative to substrate 110 as indicated by arrows 141. Beam 148 can also be rotated relative to surface normal 115. Arrow 143 represents two types of rotation: tilt changing the angle between axis 145 and normal 115; and azimuthal rotation about the normal 115. Charged particle optics 146 can also be used to configure width, shape (e.g. circular or elliptical), and focusing characteristics (e.g. converging, collimated, or diverging) of deposition beam 148. Through control of these beam attributes, e.g. by controller 170, shape, orientation, and position of an analyte deposition pattern can be controlled. Particularly, analyte deposition can be planned as shown at block 220 of FIG. 2, based on defect characterization using LEEPP imaging at block 210. Following block 220, at block 230, analyte 147 can be deposited on substrate 110 as described above.

Common ranges of beam parameters include: analyte beam currents of 1-30 pA; deposition times up to about 10 minutes; ion charge states (Z) in a range 5-30 for proteins and similar analyte macromolecules; or total deposited charge in a range 0.1-10 nC (often 0.5-2.0 nC) for Z in a range 10-20, or about 15.

Subsequent to deposition of analyte 147, at block 240 the LEEPP imaging subsystem can be used again to identify or map regions of substrate 110, e.g. which were previously defect-free, now containing one or more deposited analyte particles 147. In some analyte studies, it can be desirable to have exactly one particle 147 in a given aperture 132, so as to avoid interference between subsequent measurements on two proximate analyte particles. However, in other studies, having more than one (but not too many) analyte particles 147 in aperture 132 allows more analyte characterization data to be gathered in a given experimental time duration or over a given substrate area. Time can be a particular concern in some studies due to spontaneous deterioration of analyte particles, or a practical time duration over which temperature, pressure, or other process conditions can be maintained for substrate 110 and sample 147. Each analyte study can have a respective target range of analyte particles desirably deposited within one region. Illustrative examples of the target range can be 3-20 (multiple analyte particles, e.g. for faster studies), 1-10, or 1-1 (a single analyte particle, e.g. for minimal interference between particles 147).

With suitable analyte-containing regions identified, further analysis of analyte 147 can be performed at block 250. In some examples, the LEEPP imaging subsystem (or a separate but similar subsystem) can be operated at high magnification to acquire holograms of analyte 147, e.g. in a LEEH mode. These holograms can be used to reconstruct a spatial description of analyte 147. The disclosed technologies and environment are not limited to LEEH however, and a wide range of imaging, spectroscopic, or other analytic techniques can be applied.

Thus, the illustrated environment can also include one or more additional subsystems. In some examples, subsystem 150 can represent an alternative analyzer. In other examples, subsystem 150 can represent a cleaning tool for reducing extrinsic defects on substrate 110. In situ cleaning can be performed at optional block 215 prior to surface characterization, to improve yields of regions suitable for analyte studies. Cleaning can also be performed after analyte studies are complete, e.g. to remove analyte particles 147 as well as extrinsic defects, so as to render substrate 110 reusable for further analyte studies.

In some examples, a single controller can control and monitor all subsystems illustrated in FIG. 1 while, in other examples, multiple controllers can be used for the various subsystems, in any combination.

The disclosed technologies facilitate performance of analytic procedures of an analyte on a substrate. In examples, and with reference to FIG. 2, the analytic procedure can include blocks 235, or any one or more of blocks 230, 240, 250. As shown by arrow 255, the workflow of FIG. 2 can be repeated with a single substrate, or with different substrates.

### Example LEEPP Imaging

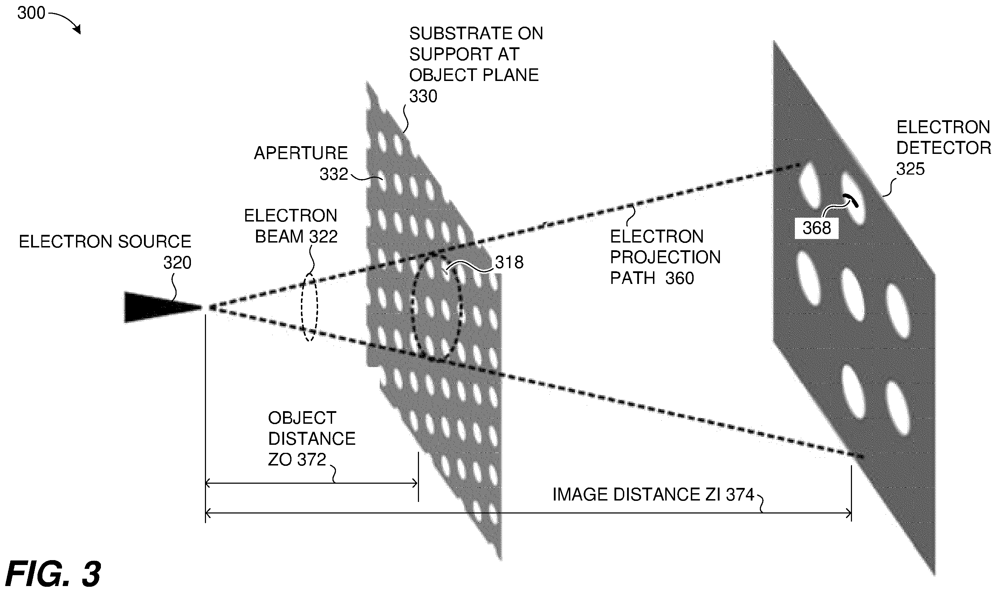

FIG. 3 is a diagram 300 illustrating an example LEEPP imaging system. Electron source 320 can be configured to emit low-energy electron beam 322 towards electron detector 325. En route, beam 322 can encounter support 330 on which substrate (similar to 110) can be mounted. Conceptually, the thin substrate can be transparent to electrons 322 and is not separately shown in FIG. 3. As shown, support 330 can be porous, having apertures 332 through which electrons 322 can propagate, while the support material itself is opaque to electrons 322. The substrate affixed to support 330 is located at an object plane of the illustrated imaging system 300.

Where able, electrons 322 propagate past support 330 along projection paths 360 toward detector 325, which is at an image plane. As shown the image formed at detector 325 can be a projection of the structure at the object plane. Illustrated in FIG. 3 is the projection of some apertures 332 within a field of view of beam 322. Similarly, image 368 of defect 318 can also be projected onto detector 325.

Measured from a tip of electron source, the substrate on support 330 is at object distance ZO 372, and detector 325 is at image distance ZI 325. According to geometric optics, the magnification M of this configuration is M = ZI/ZO. In examples, LEEPP can be performed with object distances ZO 372 about 10-50 µm and image distances ZI about 50-300 mm, or about 100-150 mm. In comparison, LEEH can be performed with object distances ZO 372 about 1 µm; a transition between geometric imaging and holographic imaging can occur around about 5 µm, for typical ZI distances.

### Examples of LEEPP Image Classes

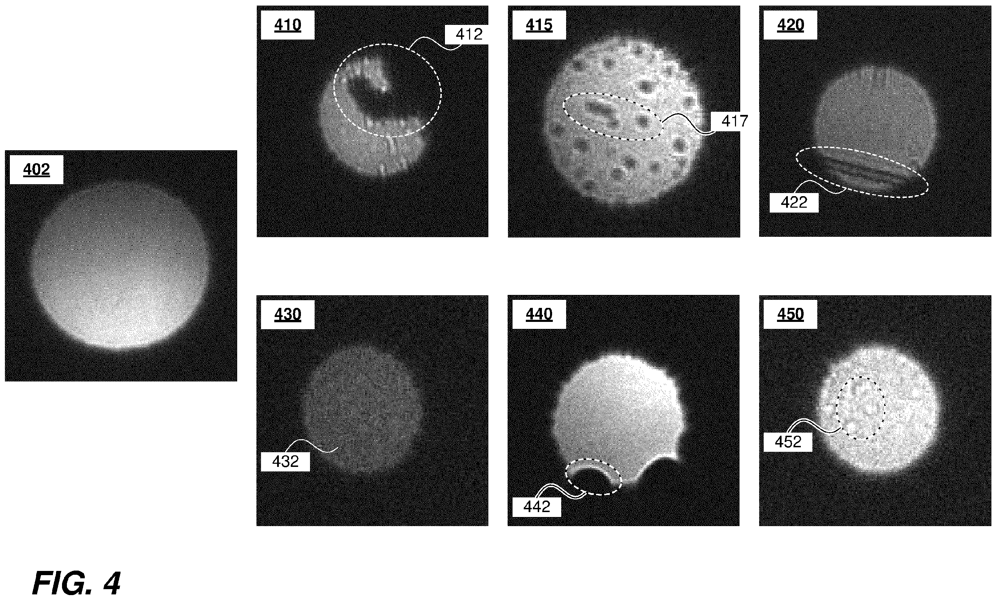

FIG. 4 is a set of LEEPP images of a graphene substrate, illustrating examples of various classes of defects. For reference, image 402 shows a defect-free region over a single aperture in a holey silicon nitride support. Images 410, 415, 420, 430, 440, 450 show a variety of exemplary defect types.

Image 410 shows a single large contaminant object 412 impeding electron transport. Contaminant 412 can be a large catalyst particle or a flake of a material used in a transfer process, e.g. to transfer a graphene substrate from a catalyst or other growth medium onto a holey silicon nitride support.

Under high magnification conditions, e.g. for LEEH imaging, object distance ZO (372) can be very small, and electric fields can be high even for the low electron energies used. Under these conditions, large contaminant 412 poses a high risk of causing arcing to the electron emitter (320). An arc can damage the electron emitter beyond repair, requiring hours or even days to replace the electron emitter. Thus, imaged region 410 can be unsuitable for analyte studies.

Image 415 shows a number of smaller defects 417, which can be fine catalyst particles on one or both surfaces of the substrate. These are also opaque to LEEPP electrons. The region image 415 has some arcing risk, although less than image 410. Particles 417 are likely to interfere with downstream holographic imaging of any analyte particle, and region 415 can also be unsuitable for analyte studies.

Image 430 shows haze on the substrate surface. While objects of significant size are not readily discernible, and arcing risk is relatively low, the haze significantly reduces electron transmission, and signal-to-noise ratio for any analyte holograms is likely to be poor.

Whereas images 410, 415, 430 show extrinsic defects, images 420, 440, 450 show examples of intrinsic defects. Image 420 shows a graphene strand 422, which can present arcing risk. Image 440 shows bright edges 442 and hole edge distortions indicative of missing graphene, which can also lead to field concentration and some relatively low risk of arcing. However, viewing conditions for analyte studies in other parts of the imaged region are good. Finally, image 450 shows a smattering of defects, sometimes termed "bubbles," indicative of a slight local accumulation of positive charge. These do not significantly impede electron transmission and do not pose significant arcing risk. For some studies, defect severity successively decreases from image 410 to 450, in order.

Different analyte studies can have different sensitivities to defects. In some LEEH studies, all images 410-450 can be regarded as unsuitable for analyte studies, with only images like 402 being suitable for analyte imaging. In other LEEH studies, image 450 can be regarded as suitable (in addition to image 402), while images 410-440 are unsuitable. In further examples, images {402, 440, 450} can be regarded as suitable, with the remaining defect examples determined to be unsuitable.

### Example Analyte Mapping Using LEEPP Imaging

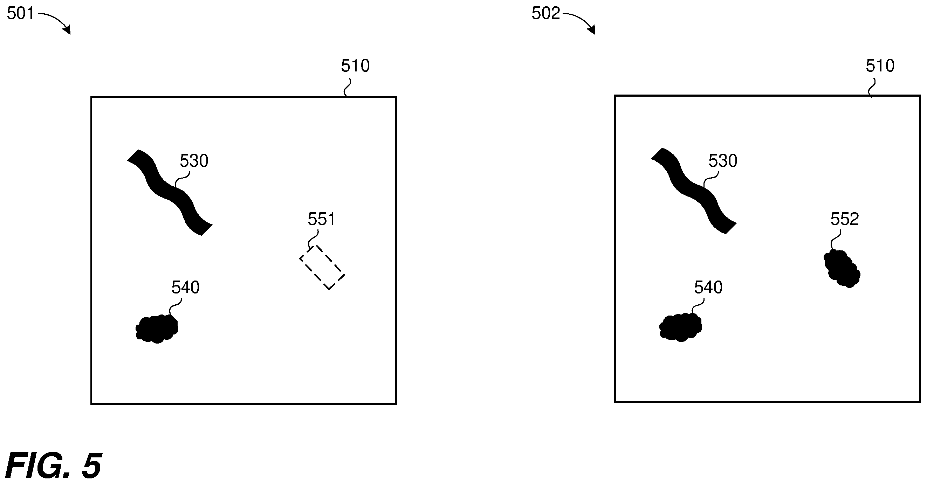

FIG. 5 shows a pair of schematic drawings 501-502 illustrating LEEPP images acquired before and after analyte deposition respectively. Drawing 501 shows a substrate 510 having a defect 530, which can be an intrinsic defect (e.g. a rupture in a graphene substrate) or an extrinsic defect (e.g. a surface contaminant). Also shown is analyte particle 540 which may have been previously deposited and characterized. Thus, drawing 501 can represent a LEEPP image taken at block 210 on a second iteration of the workflow of FIG. 2. In drawing 501, dashed outline 551 indicates a clean region on substrate 510.

Drawing 502 shows substrate 510 at a later time, after further analyte deposition, e.g. at block 240 of a third iteration of the FIG. 2 workflow. Defect 530 and earlier analyte 540 remain in place, however analyte particle 552 has been deposited and is visible in LEEPP image 502. Thus comparison of images 502 and 501 can show that newly deposited analyte molecule 552 is present at the previously clean region 551. Analyte molecule 552 can be further characterized, e.g. by LEEH imaging at block 250 of the third workflow iteration.

### Example Image Sequences

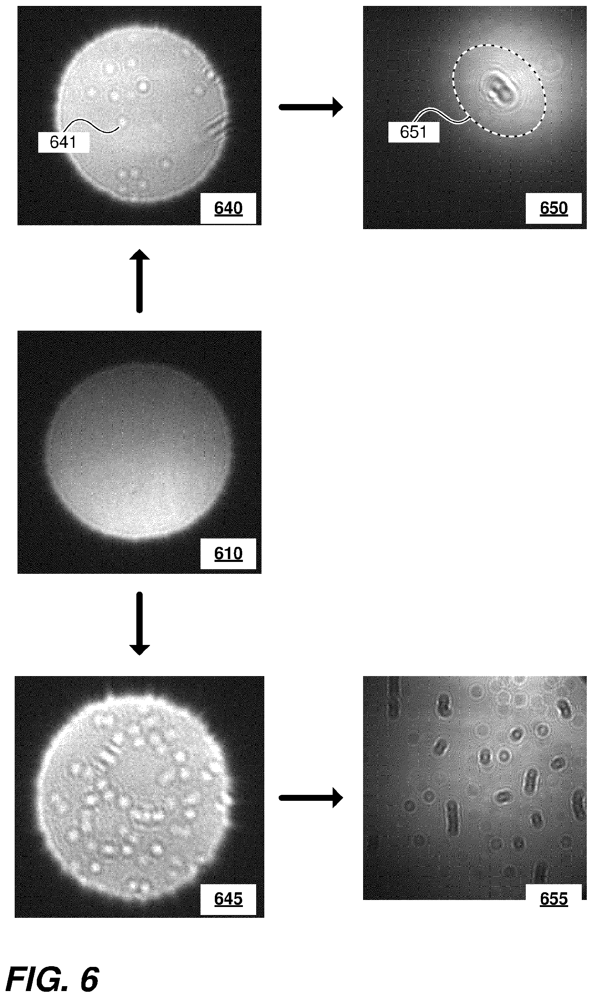

FIG. 6 is a set of example images according to the workflow of FIG. 2. Initially, at block 210, a LEEPP scan of a substrate can identify, in image 610, a defect-free region of a substrate.

In a first example, the same region can produce a second LEEPP image 640 at block 240. In image 640, between 10 and 20 protein molecules 641 are discernible. Then, a higher magnification image 650 can be obtained at block 250 using LEEH. Hologram 651 is well separated from images of other protein molecules and can be used for high quality spatial reconstruction of the protein molecule 641.

In a second example, an image similar to 645 can be obtained at block 240. Image 645 shows approximately 50 protein molecules, some of which have spontaneously clustered in chains. A corresponding LEEH image 655 can be obtained at block 250. Image 655 has lower magnification than image 650, and may be suitable for certain studies. However, at higher magnification, interference between holograms of neighboring protein molecules can increase, rendering the holograms unsuitable for protein characterization.

### Example Regions and Deposition Patterns

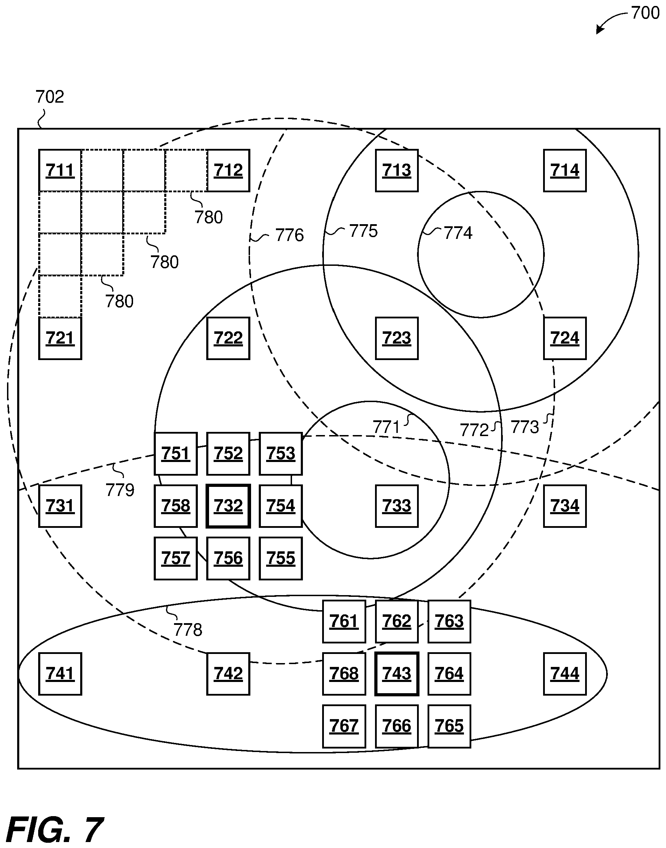

FIG. 7 is a diagram 700 of a substrate illustrating example regions and example deposition patterns according to the disclosed technologies.

The available area of substrate 702 can be subdivided into tiles such as 711-714 or 780. Initially, a sparse set of tiles 711-714, 721-724, 731-734, 741-744 can be imaged by LEEPP, while other tiles 780 can be omitted. As described herein, tile size and LEEPP magnification can be jointly selected to optimize scan efficiency. Some imaged tiles may be found to have an acceptable severity level of defects (including no visible defects), while other imaged tiles may be found to have an unacceptable severity level of defects.

The description assumes, for purpose of illustration, that tiles 732, 743 are found to have acceptable defect severity and are therefore suitable for a subsequent analytic procedure, e.g. for characterization of an analyte. Then, a secondary LEEPP imaging scan can be performed over tiles 751-758 which neighbor tile 732. Similarly, a secondary LEEPP imaging scan can be performed over tiles 761-768 which neighbor suitable tile 743. Because substrate defects, both intrinsic and extrinsic, can often be spatially correlated, the vicinity of suitable (e.g. clean) tile 732, 743 can have a greater likelihood of additional acceptable tiles as compared to the vicinity of an unsuitable (e.g. defect-laden) tile.

Also shown in FIG. 7 are several circles and ellipses, or portions thereof, which represent analyte deposition patterns. Circles 774-776 represent one such pattern. Analyte deposition density from a deposition beam similar to 148 of FIG. 1 can have a bell-shaped, e.g. Gaussian or parabolic, profile decreasing away from axis (145). Also, as described herein, a given study can have a target range of how many visible analyte particles are desirably deposited in one region (tile). Illustratively, circles 774, 775 can be the loci at which the expected analyte deposition density times tile area equals the upper or lower limits of the target range, respectively. Thus, tiles lying within the annular region bounded by circles 774, 775 have an expected number of analyte particles within the target range. Tiles lying inside circle 774 can have an expected number of analyte particles higher than desirable, which can lead to interference between high magnification holograms of proximate analyte particles. Tiles lying outside circle 775 can receive fewer analyte particles than desirable. Analyte particles in such tiles can still be characterized, however the available substrate area could be better utilized with more analyte particles. Then, another dashed line 776 indicates an extent of the deposition pattern. In some examples, circle 776 can correspond to a deposition density of 0.5 analyte particles per tile while, in other examples, delivery subsystem (146) can include a clipping aperture and circle 776 can represent a sharp edge of an instant deposition pattern.

The contour lines of deposition pattern 774-776 are shown as concentric circles, but this is not a requirement. Through control of beam shape and deposition axis tilt, other deposition patterns can be achieved. Pattern 771-773 has generally circular contours which are not concentric, while pattern 778-779 has elliptical contours which are concentric. Notably, the entire interior of ellipse 778 is within a target range for deposited analyte; there is no inner contour similar to 774 corresponding to the upper limit of desired deposition density.

As also illustrated by patterns 771-773, 774-776, 778-779, a deposition pattern can be suitably positioned across, or even off, substrate 702 so as to optimize utilization of substrate 702, according to various criteria described herein, over one or more iterations of a workflow similar to FIG. 2.

Each illustrated deposition pattern can reflect an instantaneous deposition profile of an analyte beam (148) as well as temporal position drift between source (140) and substrate (110).

### First Example Method - Identifying Suitable Regions

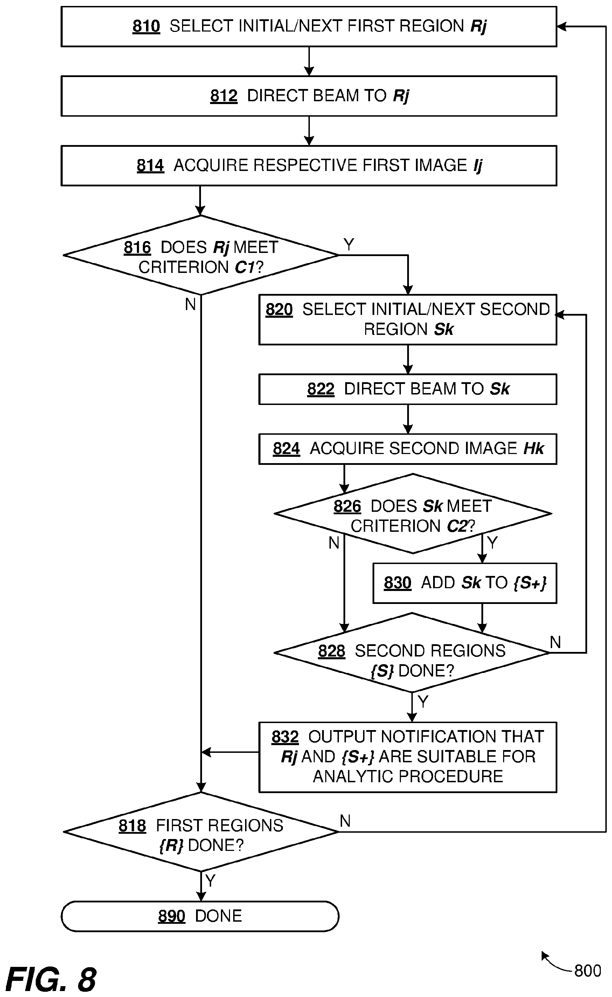

FIG. 8 is a flowchart 800 of a first example method for identifying regions suitable for a subsequent analytic procedure. In this method, imaging scans are performed in two phases to efficiently identify regions suitable for the analytic procedure over an entire substrate area. This method is described with reference to FIG. 7. This method can be used to implement block 210 of FIG. 2.

Process block 810 is the beginning of a first iterative loop, over first regions {Rj}. At block 810, an initial first region (e.g. tile 711) can be selected on a first iteration, or a next first region (e.g. tiles 712, 713, ... 743, 744) can be selected on subsequent iterations. At block 812, a beam (e.g. a low-energy electron beam similar to 122 of FIG. 1) can be directed to the instant region Rj and, at block 814, first image Ij of region Rj can be acquired.

At decision block 816, a determination can be made, based on image Ij, whether region Rj satisfies criterion C1 for performing a subsequent analytic procedure. Criterion C1 can be based on visible defects identified in image Ij. Criterion C1 can be based on types or numbers of defects. To illustrate, a single large contaminant or substrate strand may be sufficient for a region to fail criterion C1, while small contaminants not exceeding a threshold number may be acceptable, and small intrinsic defects (e.g. bubbles) may be acceptable in any number.

If region Rj is not acceptable, the method can proceed directly via the N branch to decision block 818, where a check is made whether additional regions {Rj} remain to be scanned. If all regions {Rj} have been scanned, the method can continue via the Y branch to termination block 890 and the method can terminate. Otherwise, the method can return via the N branch from block 818 to block 810 to iterate the first loop for a next region Rj.

However, if region Rj is acceptable, the method can follow the Y branch to block 820, which is the beginning of a second iterative loop, over second regions {Sk}. At block 820, an initial second region Sk (e.g. tile 751, 761) can be selected on a first iteration, or a next second region (e.g. tiles 752 ... 758, or tiles 762 ... 768) can be selected on subsequent iterations. At block 822, a beam (e.g. 122) can be directed to instant region Sk and, at block 824, second image Hk of region Sk can be acquired.

At decision block 826, a determination can be made, based on image Hk, whether region Sk satisfies criterion C2 for performing the subsequent analytic procedure. Criterion C2 can often be the same as criterion C1, but this is not a requirement.

If region Sk is not acceptable, the method can proceed directly via the N branch to decision block 828, where a check is made whether additional regions {Sk} remain to be scanned for the instant region Rj. If all regions {Sk} (e.g. tiles 751-758) have been scanned, the method can continue via the Y branch to termination block 832. Otherwise, the method can return via the N branch from block 828 to block 820 to iterate the second loop for a next region Sk.

If region Sk is acceptable, then the method can follow the Y branch from block 826 to block 830, where Sk can be added to a set {S+} of suitable second regions. Thereafter, the method continues to decision block 828, described above.

At block 830 a notification of suitable regions can be outputted. This can include instant region Rj, and all regions {S+} that satisfied criterion C2. In some cases, there may be no regions {S+} satisfying criterion C2. The notification can inform a controller or other recipient that the noted regions are suitable for a subsequent analytic procedure.

Numerous extensions or variations of the first method can be implemented within the scope of the disclosed technologies. The substrate can be graphene, e.g. a monolayer. The beam can be an electron beam, such as low-energy electron beam 122. In some examples, directing the beam at block 812 or block 822 can be performed using one or more electromagnetic deflection elements. In other examples, the substrate can be mounted on a stage (135), the beam can be produced by a source (120), and directing the beam at block 812 or block 822 can be performed by relative translation between the beam source and the stage.

In some examples, criterion C1 can be that defects visible in the respective first image Ij have severity not exceeding a first predetermined threshold. Similarly, criterion C2 can be that defects visible in the respective second image Hk have severity not exceeding a first predetermined threshold. A variety of techniques can be used for making the determinations at blocks 816 or 826. In some examples, the respective image Ij or Hk can be displayed to a user, and an input can be received from the user indicating whether region Rj or Sk meets criterion C1 or C2 respectively. In other examples, a signal can be calculated from image Ij or Sk indicating a defect severity in that image. The calculated signal can be compared with a predetermined threshold to determine whether criterion C1 or C2 is met. To illustrate, machine visions heuristics based on pixel intensity variations, edge detection, or shape detection can be used to calculate the signal. In further examples, the respective image Ij or Hk can be inputted to a trained machine learning classifier, and a corresponding output can be received. The output can indicate whether criterion C1 or C2 is met.

In further examples, the method can be extended to performing the subsequent analytic procedure on the first region Rj (which satisfies criterion C1). The subsequent analytic procedure can include deposition of an analyte species (in the form of particles or molecules of the analyte) onto the substrate over a time interval by a deposition beam having a beam axis. The beam axis and the time interval can be selected such that the suitable first region Rj lies within a predetermined zone (similar to the annulus between 771, 772 of FIG. 7, or the interior of ellipse 778) of a deposition pattern. That is, the deposition pattern can be planned (similar to block 220) so that region Rj is expected to meet the target range of analyte particles. With beam axis and time interval set, the analyte deposition can be performed (230).

In additional examples, subsequent to the analyte deposition (230), the beam (122) can be once again directed to region Rj, and a third image can be acquired. If the third image shows that region Rj still meets criterion C1, then region Rj can be reused on a subsequent iteration of the workflow of FIG. 2. If the third image shows that region Rj has at least one analyte particle, but not more than a predetermined limit, then those analyte particles can be characterized. In some examples, the third image can be a high-magnification hologram (LEEH), and hologram reconstruction can be performed to derive a fourth image from the third image, the reconstructed fourth image being used for characterizing the analyte.

In varying examples, criteria C1, C2 can be applied on a tile or aperture basis. In some examples having multiple apertures per tile image, a defect severity criterion can be applied separately to each aperture within a tile image, and a decision as to tile suitability can be made based on criteria evaluations for the various apertures. In some examples, a tile can be deemed suitable if all apertures within the tile image meet a defect severity criterion while, in other examples, a tile can be deemed suitable if at least a predetermined number of its apertures meet the defect severity criterion.

In other examples, a set of regions {Sk} scanned in the second loop (blocks 820-828) can also be sparse. The second loop can be performed multiple times for a single region Rj, with different regions {Sk}, to progressively identify additional suitable regions.

In further examples, a controller (170) can be configured to perform the first method or any of its variations or extensions, and the disclosed technologies can be embodied in an apparatus including the controller, a beam source, and a detector. The beam source can be configured to direct the beam, as controlled by the controller at blocks 812, 822, to a selected portion of the substrate. The detector can be configured to image the selected portion of the substrate illuminated by the beam. The detector can incorporate a pixelated electron detector.

### Second Example Method - Analyte Deposition

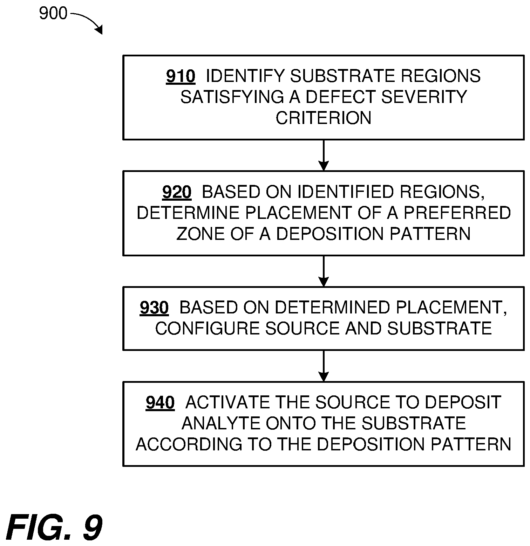

FIG. 9 is a flowchart 900 of a second example method, for analyte deposition. In this method, a deposition pattern is placed based on defect severity in various regions of a substrate and analyte is deposited according to this deposition pattern. This method can be used to implement blocks 220, 230 of FIG. 2.

At process block 910, substrate regions that satisfy a defect severity criterion can be identified. In varying examples, as discussed herein, the defect severity criterion can require that a region be free of all visible defects, or can permit certain types or numbers of visible defects.

As described herein, a deposition pattern can have a preferred zone, in which the expectation value of a number of deposited analyte particles is within a predetermined target range, while regions outside the preferred zone are expected to have too many or too few deposited particles. At block 920, the placement of a preferred zone of a deposition pattern can be determined so that at least one of the regions identified at block 910 lie within the preferred zone. A region can be deemed to lie within the preferred zone if a centroid of the region lies within the preferred zone.

At block 930, the combination of source and substrate can be configured based on the placement determined at block 920. In varying examples, this can involve configuring just the source, just the substrate, or both the source and the substrate. For example, position or orientation of the source or the substrate can be adjusted. Additionally, source parameters including time duration, intensity, size, or shape of a deposition beam can be configured.

Then, at block 940, the source can be activated to deposit analyte onto the substrate according to the deposition pattern determined at block 920.

Numerous extensions or variations of the first method can be implemented within the scope of the disclosed technologies. The analyte can include a protein. The identifying can include performing a sparse scan of substrate regions, similar to 711-714, 721-724, 731-734, 741-744 of FIG. 7. To illustrate, 4 µm × 4 µm tiles can be imaged on a 25 µm × 25 µm grid over a 500 µm × 500 µm substrate, thereby imaging 400 tiles out of 15,625 tiles.

The configuring at block 930 can include adjusting distance (149) between the source and substrate, adjusting an analyte emission rate of source (140, 142), adjusting a time duration of analyte deposition, or adjusting an angle (143) between the deposition axis (145) and a surface normal (115) of the substrate. The target range defining the preferred zone can lie between a first threshold and a second threshold. Example target ranges can include 1-1, 1-2, 1-10, 2-5, 2-10, 3-10, 3-20, 3-30, 5-10, 5-20, 5-30, 10-20, 10-30, 10-50, or 20-50. Deposition axis (145) can be aimed at a portion of substrate (110) having a highest density of suitable regions, or at a centroid of the suitable regions.

In examples, the determining at block 920 can optimize a number of identified regions (e.g. satisfying a defect severity criterion) lying within the preferred zone, e.g. to maximize this number. To illustrate, a pattern placement having 12 identified regions within the preferred zone can be chosen over another pattern placement having only 2 identified regions within the preferred zone. A variety of factors can be considered at block 920. First, a number of identified regions within the preferred zone can be used. Having a greater number of identified regions within the preferred zone can result in more deposited analyte particles suitable for characterization downstream, e.g. at block 250. Second, a supply of analyte may be limited, and a deposition pattern that consumes less of the analyte supply can be preferred over another deposition pattern that consumes more analyte. Accordingly, a second factor that can be considered is a total amount of analyte consumed at the source (142) during the analyte deposition.

Then, analyte deposition may leave some identified regions without any analyte particles, e.g. if such regions are outside the deposition pattern, and these regions may be available for reuse, e.g. on another iteration of the workflow of FIG. 2. Accordingly, a third factor that can be considered at block 920 is an expected second number of the identified regions what will (still) satisfy the defect severity criterion after the analyte deposition. To illustrate with reference to FIG. 7, consider a scenario with regions 714, 721, 723, 732 identified as defect-free. Then, pattern 774-776 includes 2 regions 714, 723 within the preferred zone, while regions 721, 732 are outside pattern extent 776, and hence may be available for use on a subsequent workflow iteration. Alternative pattern 771-773 also includes two regions 723, 732 within the preferred zone, but only one identified region 714 lies outside perimeter 773. Inasmuch as region 721 lies within perimeter 773, it may receive a sub-optimal number of analyte particles on a current workflow iteration, but may also not be suitable for use on a subsequent workflow iteration. Thus, although both patterns 774-776 and 771-773 provide the same number (2) of identified regions within the preferred zone, the former can be preferred because of a greater availability of regions (2 vs 1) likely available for later use.

Optimization can be performed in various ways. Some parameters can be held fixed and other parameters can be varied. In some examples, e.g. where variable parameters are few, optimization can be performed by scanning parameter values over multiple configurations and, for each configuration, predicting outcomes for various factors, such as counts of deposited analyte particles in the identified regions. Then, one among the scanned configurations can be selected as being optimal for the factor(s) under consideration. Parameter values can be scanned monotonically, using a binary search, random selection, or a combination of these techniques. In other examples, a cost function can be defined based on the factor(s) under consideration, and parameters can be varied to optimize the cost function. A gradient descent or steepest descent technique can be used.

In further examples, a controller (170) can be configured to perform the second method or any of its variations or extensions, and the disclosed technologies can be embodied in a system including the controller and the source (140). The deposition source can include an electro-spray ionizer (142), a mass spectrometer (144), and charged particle optics (146). The ionizer can be coupled to deliver first ionized analyte molecules to an input of the mass spectrometer. The charged particle optics can be coupled to receive a fraction of the first ionized analyte molecules, e.g. filtered by mass-to-charge ratio, and can be configured to deposit the second analyte molecules on the substrate.

### Third Example Method - LEEPP Image Classification

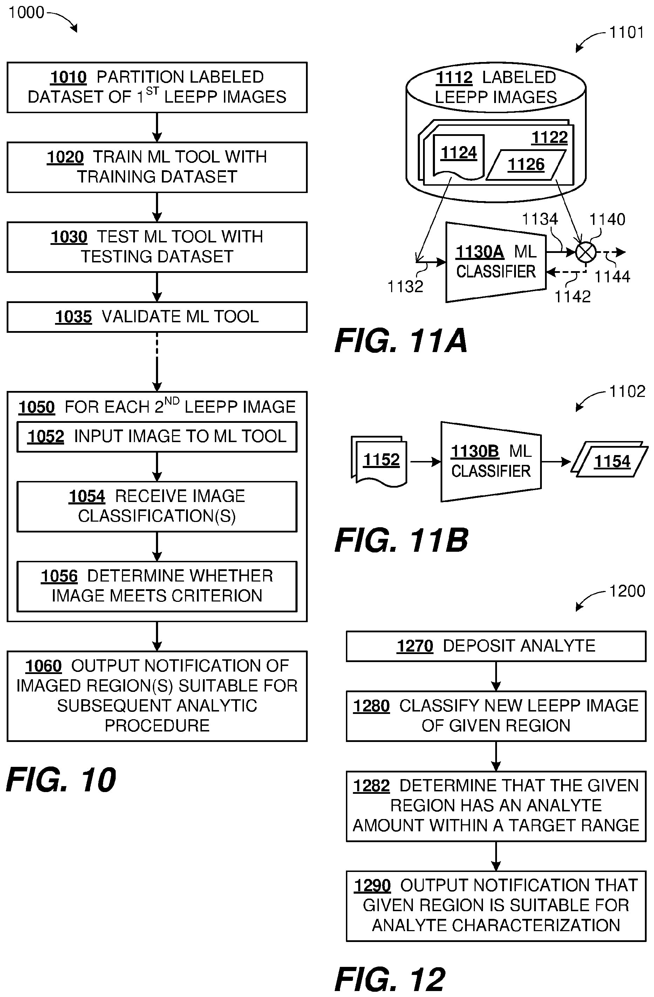

FIG. 10 is a flowchart 1000 of a third example method which employs a trained machine learning (ML) tool to classify LEEPP substrate images. FIGS. 11A-11B provide dataflow diagrams 1101-1102 for respective phases of the method of FIG. 10, and are used to facilitate discussion of FIG. 10. Portions of this method can be used to implement blocks 210, 240 of FIG. 2; 816, 826 of FIG. 8; or block 910 of FIG. 9.

In FIG. 10, blocks 1010-1035 constitute a training phase of ML classifier 1130A, an objective of which is to have classifier 1130A validated for classification of LEEPP substrate images, for example with respect to defect severity. Classifiers 1130A of FIG. 11A and 1130B of FIG. 11B can represent a same classifier, before and after validation respectively.

At process block 1010, a labeled dataset 1112 can be partitioned into a training dataset and a testing dataset. Each record 1122 of dataset 1112 can include a LEEPP image 1124 and one or more classifications 1126 assigned by one or more users (e.g. human experts). The user-assigned classification(s) 1126 can be indicative of visible defects in image 1124 selected from a plurality of predetermined classifications. In some examples, each image 1124 can span a single aperture (132), but this is not a requirement and, in other examples, classifier 1130A can be trained on tile images, each encompassing multiple apertures.

At block 1020, ML tool 1130A can be trained using the training dataset. Illustratively, successive images 1124 can provided as input 1132 to trainee tool 1130A, and corresponding output classifications 1134 can be received. Outputs 1134 can be compared with the corresponding user-assigned classifications 1126 by software module 1140 to obtain a loss function 1142 which can be fed back to ML tool 1130A for updating coefficients within tool 1130A, e.g. by backpropagation.

Then, at block 1030, the testing dataset can be applied to tool 1130A to generate test classifications. To this point, training has been applied to tool 1130A, but tool 1130A has not been validated. Illustratively, testing images 1124 can be provided as input 1132 to trained tool 1130A, and corresponding test classifications 1134 can be received. At block 1135, software module 1140 (which can be same as that used at block 1020, or different) can compare the test classifications with the user-assigned classifications 1126, and the resulting comparisons 1144 can be used to validate the trained ML tool 1130A.

Blocks 1050, 1060 constitute an inference phase, in which trained ML classifier 1130B can be used to characterize LEEPP images of substrate regions. FIG. 11B shows a corresponding dataflow. As indicated by block 1050, common processing of blocks 1052, 1054, 1056 can be applied to multiple images 1152 of respective substrate regions. At block 1052, an instant image 1152 can be input to trained ML classifier 1130B, and at block 1154 one or more corresponding image classifications can be received. Then, at block 1056, a determination can be made based on the one of more classifications whether the respective substrate region of the current image meets a criterion for a subsequent analytic procedure. For purpose of illustration, the description considers a case where at least a given one of the imaged substrate regions does meet the criterion. Then, at block 1060, a notification can be issued indicating that the given substrate region is suitable for the subsequent analytic procedure.

Numerous extensions or variations of the first method can be implemented within the scope of the disclosed technologies. The subsequent analytic procedure can include the group of operations 235 of FIG. 2, or any one or more of operations 230, 240, 250. The ML classifier can be implemented as a convolutional neural network.

In one example, ML classifier 1130A, 1130B was implemented as a CNN having 23 convolutional layers and 3 fully connected ("FC") layers, and operating on 128×128 pixel images. A labeled dataset 1112 having about 7000 records 1122 was used for training. Other CNNs having 10-50 convolutional layers and 2-10 fully connected layers can also be used, operating on input image sizes 32×32 to 1024×1024 pixels.

Commensurate with labels 1126 applied in training dataset, classifications 1154 can be organized in numerous ways. In some examples, classifications 1154 can be binary: either a given image is suitable or unsuitable for the analytic procedure. As described herein, various criteria can be used for suitability, such as: the image is completely free of visible defect; the image has at most certain benign classes of defect; or the image has at most a limit number of some other class of defects; or combinations thereof. In other examples, more fine-grained classifications can be used, such as: the image is free of visible defects; at least one intrinsic defect is present; or at least one extrinsic defect is present. Additional or alternative classifications can be used to denote presence of at least one analyte molecule, or whether a number of analyte molecules is below a target range, within the target range, or above the target range. Alternatively, classification can indicate the most severe type of defect present, e.g. among classes of defects described in context of FIG. 4. Still further, a single image can receive multiple classifications, e.g. separate classifications indicating presence of both intrinsic and extrinsic defects; or binary present/absent classifications for each of multiple types of defect.

In some examples, LEEPP images can be acquired in tiles spanning multiple apertures (similar to 132), whereas classification can be performed on smaller regions containing a single aperture. Thus, prior to block 1052, acquired images can be split into the individual images operated on by block 1050. Machine vision techniques can be used to identify circular areas of individual apertures from surrounding low intensity regions corresponding to an opaque support, as seen in FIGS. 3-4.

FIG. 12 is a flowchart 1200 of another example extension to the method of FIG. 10. In this extension, the classifier is used for mapping analyte deposition, e.g. at block 240 of the workflow of FIG. 2. At block 1270 (similar to block 230), analyte deposition can be performed as all or part of the subsequent analytic procedure, over at least a given region that was determined, at block 1056, to be suitable for the analytic procedure. Thereafter, at block 1280, a new LEEPP image of the given region can be classified by trained ML classifier 1130B and, at block 1282, a determination can be made that the given region contains a number of the analyte molecules within a target range. In some examples, the classification received at block 1280 can directly indicate that the number of analyte molecules is within the target range while, in other examples, the classification at block 1280 can indicate that analyte molecules are present, and these can be counted, e.g. using machine vision techniques for shape detection, at block 1282. Then, at block 1290, a notification can be outputted indicating that the given region is suitable for characterization of one or more analyte molecules. As described herein, this characterization can be performed by LEEH, but this is not a requirement and other analyses can also be performed.

### Additional Examples

The following are additional examples of the disclosed technologies.

Example 1 is a computer-implemented method, including: directing a beam successively to a plurality of first regions of a substrate; for each of the first regions: acquiring a respective first image; and based on the respective first image, determining whether the first region meets a first criterion for a subsequent analytic procedure; and wherein at least a given one of the first regions meets the first criterion; for the given first region: directing the beam successively to a plurality of second regions neighboring the given first region; for each of the second regions: acquiring a respective second image; and based on the respective second image, determining whether the second region meets a second criterion for the subsequent analytic procedure; and outputting a notification that the given first region, and all of the second regions meeting the first criterion, are suitable for the subsequent analytic procedure.

Example 2 includes the subject matter of Example 1, and further specifies that one or more of the directing actions comprise steering the beam with one or more electromagnetic deflection elements.

Example 3 includes the subject matter of any of Examples 1-2, and further specifies that the beam is produced by a beam source, the substrate is mounted on a stage, and one or more of the directing acts comprise performing relative translation of the beam source and the stage.

Example 4 includes the subject matter of any of Examples 1-3, and further specifies that the first criterion is that defects visible in the respective first image have severity not exceeding a first predetermined threshold.

Example 5 includes the subject matter of any of Examples 1-4, and further specifies that the second criterion is that defects visible in the respective second image have severity not exceeding a second predetermined threshold.

Example 6 includes the subject matter of any of Examples 1-5, and further specifies that the determining whether the first region meets the first criterion comprises: (a) displaying the respective first image to a user and receiving an input from the user indicating whether the first region meets the first criterion; (b) calculating a signal indicating a defect severity in the respective first image, and comparing the calculated signal with a predetermined threshold, a result of the comparing indicating whether the first region meets the first criterion; or (c) providing the respective first image as input to a trained classifier and receiving a corresponding output from the trained classifier indicating whether the first region meets the first criterion.

Example 7 includes the subject matter of any of Examples 1-6, and further includes: performing the subsequent analytic procedure on the given first region.

Example 8 includes the subject matter of Example 7, and further specifies that the subsequent analytic procedure comprises deposition of an analyte species over a time interval by a deposition beam having a beam axis, and the performing further comprises: selecting the beam axis and the time interval such that the given first region lies in a predetermined zone of a deposition pattern; and activating the deposition beam to deposit the analyte species onto the substrate.

Example 9 includes the subject matter of any of Examples 7-8, and further specifies that the subsequent analytic procedure comprises deposition of an analyte species and the method further comprises, subsequent to the performing: directing the beam to the given first region; acquiring a third image of the given first region; and determining whether the third image meets the first criterion; determining whether the third image shows presence of a number of analyte particles in a range from one to a predetermined limit; or outputting a fourth image derived from the third image to be used for characterizing the analyte species.

Example 10 includes the subject matter of any of Examples 1-9, and further specifies that the substrate is graphene.

Example 11 is one or more computer-readable media storing instructions which, when executed by one or more hardware processors, cause the one or more hardware processors to perform the method of any of Examples 1-10.

Example 12 is an apparatus, including: a controller having memory coupled thereto and configured to perform the method of any of Examples 1-10; a beam source configured to direct the beam to a selected portion of the substrate; and a detector configured to image the selected portion illuminated by the beam.

Example 13 includes the subject matter of Example 12, and further specifies that the beam source is an electron source and the beam is an unfocused electron beam having energy between 50 eV and 600 eV.

Example 14 includes the subject matter of any of Examples 12-13, and further specifies that the detector comprises a pixelated electron detector.

Example 15 is a computer-implemented method of controlling analyte deposition on a substrate by a source having a deposition axis, the method comprising: identifying a plurality of regions of the substrate satisfying a defect severity criterion; determining placement of a preferred zone of a deposition pattern on the substrate so that at least one of the identified regions lies within the preferred zone; configuring a combination of the source and the substrate according to the determined placement; and activating the source to deposit analyte onto the substrate according to the deposition pattern.

Example 16 includes the subject matter of Example 15, and further specifies that the analyte comprises a protein.

Example 17 includes the subject matter of any of Examples 15-16, and further specifies that the identifying comprises performing a sparse scan over the substrate.

Example 18 includes the subject matter of any of Examples 15-17, and further specifies that the configuring comprises: relative translation of the source and a stage supporting the substrate; or relative rotation of the deposition axis and the stage supporting the substrate.

Example 19 includes the subject matter of any of Examples 15-18, and further specifies that the configuring comprises controlling the deposition pattern by adjusting one or more parameters including: distance between the source and the substrate; an analyte emission rate of the source; a time duration of the analyte deposition; or an angle between the deposition axis and a surface normal of the substrate.

Example 20 includes the subject matter of any of Examples 15-19, and further specifies that: a region within the preferred zone has a first probability of receiving a number of analyte molecules within a predetermined range, during the analyte deposition, the first probability being greater than or equal to a first threshold and less than or equal to a second threshold; and regions outside the preferred zone have respective probabilities of receiving respective numbers of the analyte molecules within the predetermined range, during the analyte deposition, which are less than or equal to the first threshold or greater than or equal to the second threshold.

Example 21 includes the subject matter of any of Examples 15-20, and further specifies that the determining optimizes a number of the identified regions that lie within the preferred zone.

Example 22 includes the subject matter of any of Examples 15-21, and further specifies that factors used in the determining comprise: a first number of the identified regions that lie within the preferred zone; an expectation value of a second number of the identified regions that will satisfy the defect severity criterion after the analyte deposition; or a total amount of analyte consumed at the source during the analyte deposition.

Example 23 is a system, including: a controller having memory coupled thereto and configured to perform the method of any of Examples 15-22; and the source.

Example 24 includes the subject matter of Example 23, and further specifies that the source comprises: a mass spectrometer; an ionization source coupled to deliver the analyte, in charged particles, to an input of the mass spectrometer; and charged particle optics coupled to receive ionized analyte molecules having a selected mass-to-charge ratio from an output of the mass spectrometer, and configured to deposit the second analyte molecules on the substrate.

Example 25 is a method comprising: during a training phase: partitioning a labeled dataset comprising a plurality of records into a training dataset and a testing dataset, each record comprising a first low-energy electron point projection (LEEPP) image of a respective substrate region and one or more respective user-assigned classifications, the user-assigned classifications selected from among a plurality of predetermined classifications and indicative of defects visible in the respective first LEEPP image; training a machine learning tool with the training dataset; applying the testing dataset to the trained machine learning tool to generate test classifications; and comparing the test classifications with the respective user-assigned classifications to validate the trained machine learning tool; and during characterization of a given substrate: for each of a plurality of second LEEPP images of a respective region of the given substrate: providing the respective second LEEPP image to the trained machine learning tool; receiving, from the trained machine learning tool, one or more of the predetermined classifications; and determining, based on the received classification(s), whether the respective region of the given substrate meets a criterion for a subsequent analytic procedure; wherein at least a given one of the regions of the given substrate meets the criterion; and outputting a notification that the given region is suitable for the subsequent analytic procedure.

Example 26 includes the subject matter of Example 25, and further specifies that the machine learning tool is a convolutional neural network (CNN), vision transformer neural network (ViT), or other neural network.

Example 27 includes the subject matter of any of Examples 25-26, and further specifies that the predetermined classifications are binary, with two classifications respectively indicating that a corresponding substrate region is suitable or unsuitable for the subsequent analytic procedure.

Example 28 includes the subject matter of any of Examples 25-27, and further specifies that the predetermined classifications include at least: a first classification indicating that a corresponding substrate region is free of defects; a second classification indicating presence of at least one intrinsic defect; and a third classification indicating presence of at least one extrinsic defect.

Example 29 includes the subject matter of any of Examples 25-28, and further specifies that the predetermined classifications include at least: a first classification indicating that a corresponding substrate region is free of defects; and a plurality of additional classifications indicating presence of respective grades of intrinsic defects, or presence of respective grades of extrinsic defects.

Example 30 includes the subject matter of any of Examples 25-29, and further specifies that: the subsequent analytic procedure comprises deposition of analyte molecules; and the predetermined classifications include at least: a first classification indicating that a corresponding substrate region is free of defects; a second classification indicating presence of at least one defect; and a third classification indicating presence of at least one of the analyte molecules.

Example 31 includes the subject matter of any of Examples 25-30, and further includes, prior to the providing: splitting acquired LEEPP images of the given substrate, each spanning a plurality of the respective regions, into the second LEEPP images, which span one region each.

Example 32 includes the subject matter of any of Examples 25-31, and further specifies that the subsequent analytic procedure comprises deposition of analyte molecules, and the method further comprises: subsequent to the deposition, during an analyte detection phase: classifying, by the trained machine learning tool, a new LEEPP image of the given region to obtain a new classification; determining, based on the new classification, that the given region contains a number of the analyte molecules within a target range; and outputting a notification that the given region is suitable for characterization of one or more analyte molecules.

Example 33 is a system, including: one or more hardware processors with memory coupled thereto: computer-readable memory storing instructions which, when executed by the one or more hardware processors, cause the one or more hardware processors to perform the method of any of Examples 25-32.

### A Generalized Computer Environment

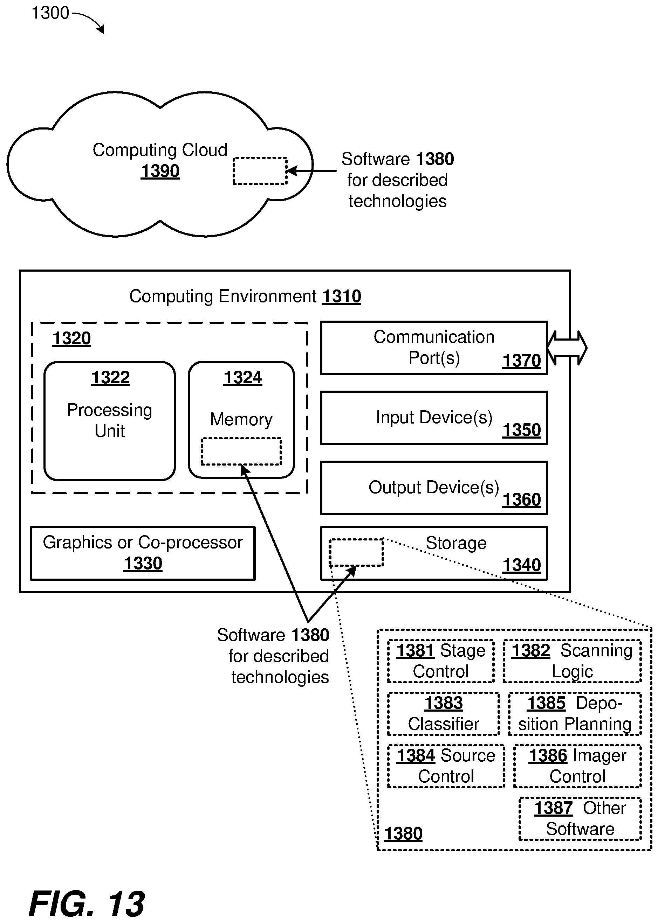

FIG. 13 illustrates a generalized example of a suitable computing system 1300 in which described examples, techniques, and technologies for substrate classification and analytic procedures can be implemented. The computing system 1300 is not intended to suggest any limitation as to scope of use or functionality of the present disclosure, as the innovations can be implemented in diverse general-purpose or special-purpose computing systems. The computing system 1300 can control or monitor an electron point projection imager, a stage, an analyte source, an analyzer, or auxiliary equipment; or can acquire, process, output, or store configuration, measurement, classification, or characterization data.

With reference to FIG. 13, the computing environment 1310 includes one or more processing units 1322 and memory 1324. In FIG. 13, this basic configuration 1320 is included within a dashed line. Processing unit 1322 can execute computer-executable instructions, such as for control or data acquisition as described herein. Processing unit 1322 can be a general-purpose central processing unit (CPU), a processor in an application-specific integrated circuit (ASIC), or any other type of processor. In a multi-processing system, multiple processing units execute computer-executable instructions to increase processing power. Computing environment 1310 can also include a graphics processing unit or co-processing unit 1330. Tangible memory 1324 can be volatile memory (e.g., registers, cache, or RAM), non-volatile memory (e.g., ROM, EEPROM, or flash memory), or some combination thereof, accessible by processing units 1322, 1330. The memory 1324 stores software 1380 implementing one or more innovations described herein, in the form of computer-executable instructions suitable for execution by the processing unit(s) 1322, 1330. For example, software 1380 can include software 1386 for controlling an imager, software 1381 for stage control, software 1382 for scanning logic, software 1383 for performing classification, software 1385 for deposition pattern planning, software 1384 for controlling an analyte source, or other software 1387 (including user interface, host interface, or hologram reconstruction). The inset shown for software 1380 in storage 1340 can be equally applicable to software 1380 elsewhere in FIG. 13. The memory 1324 can also store control parameters, calibration data, measurement data, a spectroscopic library, training data, or other database data. The memory 1324 can also store configuration or operational data.

A computing system 1310 can have additional features, such as one or more of storage 1340, input devices 1350, output devices 1360, or communication ports 1370. An interconnection mechanism (not shown) such as a bus, controller, or network interconnects the components of the computing environment 1310. Typically, operating system software (not shown) provides an operating environment for other software executing in the computing environment 1310, and coordinates activities of the components of the computing environment 1310.

The tangible storage 1340 can be removable or non-removable, and includes magnetic disks, magnetic tapes or cassettes, CD-ROMs, DVDs, or any other medium which can be used to store information in a non-transitory way and which can be accessed within the computing environment 1310. The storage 1340 stores instructions of the software 1380 (including instructions and/or data) implementing one or more innovations described herein. Storage 1340 can also store image data (including LEEPP or LEEH images), measurement data, classification data, training data, reference data, calibration data, configuration data, sample data, or other databases or data structures described herein.

The input device(s) 1350 can be a mechanical, touch-sensing, or proximity-sensing input device such as a keyboard, mouse, pen, touchscreen, or trackball, a voice input device, a scanning device, or another device that provides input to the computing environment 1310. The output device(s) 1360 can be a display, printer, speaker, optical disk writer, or another device that provides output from the computing environment 1310. Input or output can also be communicated to/from a remote device over a network connection, via communication port(s) 1370.

The communication port(s) 1370 enable communication over a communication medium to another computing entity. The communication medium conveys information such as computer-executable instructions, audio or video input or output, or other data in a modulated data signal. A modulated data signal is a signal that has one or more of its characteristics set or changed in such a manner as to encode information in the signal. By way of example, and not limitation, communication media can use an electrical, optical, RF, acoustic, or other carrier.

A data acquisition system can be integrated into computing environment 1310, either as an input device 1350 or coupled to a communication port 1370, and can include analog-to-digital converters or connections to an instrumentation bus. An instrumentation control system can be integrated into computing environment 1310, either as an output device 1360 or coupled to a communication port 1370, and can include digital-to-analog converters, switches, or connections to an instrumentation bus.

In some examples, computer system 1300 can also include a computing cloud 1390 in which instructions implementing all or a portion of the disclosed technology are executed. Any combination of memory 1324, storage 1340, and computing cloud 1390 can be used to store software instructions and data of the disclosed technologies.

The present innovations can be described in the general context of computer-executable instructions, such as those included in program modules, being executed in a computing system on a target real or virtual processor. Generally, program modules or components include routines, programs, libraries, objects, classes, components, data structures, etc. that perform particular tasks or implement particular data types. The functionality of the program modules can be combined or split between program modules as desired in various embodiments. Computer-executable instructions for program modules can be executed within a local or distributed computing system.

The terms "computing system," "computing environment," and "computing device" are used interchangeably herein. Unless the context clearly indicates otherwise, neither term implies any limitation on a type of computing system, computing environment, or computing device. In general, a computing system, computing environment, or computing device can be local or distributed, and can include any combination of special-purpose hardware and/or general-purpose hardware and/or virtualized hardware, together with software implementing the functionality described herein.

### General Considerations

As used in this application and in the claims, the singular forms "a," "an," and "the" include the plural forms unless the context clearly dictates otherwise. Additionally, the term "includes" means "comprises." Further, the term "coupled" does not exclude the presence of intermediate elements between the coupled items. Furthermore, as used herein, the terms "or" and "and/or" mean any one item or combination of items in the phrase. Terms joined by "or" or "and/or" need not be mutually exclusive.

The systems, methods, and apparatus described herein should not be construed as limiting in any way. Instead, the present disclosure is directed toward all novel and non-obvious features and aspects of the various disclosed embodiments, alone and in various combinations and sub-combinations with one another. The disclosed systems, methods, and apparatus are not limited to any specific aspect or feature or combinations thereof, nor do the disclosed systems, methods, and apparatus require that any one or more specific advantages be present or problems be solved. The technologies from any example can be combined with the technologies described in any one or more of the other examples. Any theories of operation are to facilitate explanation, but the disclosed systems, methods, and apparatus are not limited to such theories of operation.

Although the operations of some of the disclosed methods are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed systems, methods, and apparatus can be used in conjunction with other systems, methods, and apparatus. Additionally, the description sometimes uses terms like "acquire," "activate," "analyze," "apply," "calculate," "characterize," "compare," "configure," "deliver," "deposit," "determine," "direct," "display," "establish," "generate," "identify," "indicate," "obtain," "output," "partition," "plan," "produce," "provide," "receive," "repeat," "rotate," "scan," "select," "steer," "train," "translate," "transmit," "use," or "validate" to describe the disclosed techniques. These terms are high-level abstractions of the actual operations that are performed. The actual operations that correspond to these terms may vary depending on the particular implementation and are readily discernible by one of ordinary skill in the art having this disclosure at hand.

In some examples, values, procedures, or apparatus may be referred to as "lowest", "best", "maximum," "optimum," "extremum," or the like. It will be appreciated that such descriptions are intended to indicate that a selection among a few or among many alternatives can be made, and such selections need not be lower, better, less, or otherwise preferable to other alternatives not considered.

Theories of operation, scientific principles, or other theoretical descriptions presented herein in reference to the apparatus or methods of this disclosure have been provided for the purposes of better understanding and are not intended to be limiting in scope. The apparatus and methods in the appended claims are not limited to those apparatus and methods that function in the manner described by such theories of operation.

Any of the disclosed methods can be controlled by, or implemented as, computer-executable instructions or a computer program product stored on one or more computer-readable storage media, such as tangible, non-transitory computer-readable storage media, and executed on a computing device (e.g., any available computing device, including tablets, smart phones, or other mobile devices that include computing hardware). Tangible computer-readable storage media are any available tangible media that can be accessed within a computing environment (e.g., one or more optical media discs such as DVD or CD, volatile memory components (such as DRAM or SRAM), or nonvolatile memory components (such as flash memory or hard drives)). By way of example, and with reference to FIG. 13, computer-readable storage media include memory 1324, and storage 1340. The terms computer-readable media or computer-readable storage media do not include signals and carrier waves. In addition, the terms computer-readable media or computer-readable storage media do not include communication ports (e.g., 1370).

Any of the computer-executable instructions for implementing the disclosed techniques as well as any data created and used during implementation of the disclosed embodiments can be stored on one or more computer-readable storage media. The computer-executable instructions can be part of, for example, a dedicated software application or a software application that is accessed or downloaded via a web browser or other software application (such as a remote computing application). Such software can be executed, for example, on a single local computer (e.g., any suitable commercially available computer) or in a network environment (e.g., via the Internet, a wide-area network, a local-area network, a client-server network, a cloud computing network, or other such network) using one or more network computers.

For clarity, only certain selected aspects of the software-based implementations are described. Other details that are well known in the art are omitted. For example, it should be understood that the disclosed technology is not limited to any specific computer language or program. For instance, the disclosed technology can be implemented by software written in Adobe Flash, C, C++, C#, Curl, Dart, Fortran, Java, JavaScript, Julia, Lisp, Matlab, Octave, Perl, Python, Qt, R, Ruby, Rust, SAS, SPSS, SQL, WebAssembly, any derivatives thereof, or any other suitable programming language, or, in some examples, markup languages such as HTML or XML, or with any combination of suitable languages, libraries, and packages. Likewise, the disclosed technology is not limited to any particular computer or type of hardware. Certain details of suitable computers and hardware are well known and need not be set forth in detail in this disclosure.

Furthermore, any of the software-based embodiments (comprising, for example, computer-executable instructions for causing a computer to perform any of the disclosed methods) can be uploaded, downloaded, side-loaded, or remotely accessed through a suitable communication means. Such suitable communication means include, for example, the Internet, the World Wide Web, an intranet, software applications, cable (including fiber optic cable), magnetic communications, electromagnetic communications (including RF, microwave, infrared, and optical communications), electronic communications, or other such communication means.

In view of the many possible embodiments to which the principles of the disclosed subject matter may be applied, it should be recognized that the illustrated embodiments are only preferred examples of the disclosed subject matter and should not be taken as limiting the scope of the claims. Rather, the scope of the claimed subject matter is defined by the following claims. We therefore claim all that comes within the scope and spirit of these claims.

## Claims

1. A computer-implemented method, comprising:
directing a beam successively to a plurality of first regions of a substrate;
for each of the first regions:
acquiring a respective first image; and
based on the respective first image, determining whether the first region meets a first criterion for a subsequent analytic procedure; and
wherein at least a given one of the first regions meets the first criterion;
for the given first region:
directing the beam successively to a plurality of second regions neighboring the given first region;
for each of the second regions:
acquiring a respective second image; and
based on the respective second image, determining whether the second region meets a second criterion for the subsequent analytic procedure; and
outputting a notification that the given first region, and all of the second regions meeting the first criterion, are suitable for the subsequent analytic procedure.

2. The computer-implemented method of claim 1, wherein one or more of the directing actions comprise steering the beam with one or more electromagnetic deflection elements.

3. The computer-implemented method of any one of claims 1-2, wherein the beam is produced by a beam source, the substrate is mounted on a stage, and one or more of the directing acts comprise performing relative translation of the beam source and the stage.

4. The computer-implemented method of any one of claims 1-3, wherein the first criterion is that defects visible in the respective first image have severity not exceeding a first predetermined threshold.

5. The computer-implemented method of any one of claims 1-4, wherein the second criterion is that defects visible in the respective second image have severity not exceeding a second predetermined threshold.

6. The computer-implemented method of any one of claims 1-5, wherein the determining whether the first region meets the first criterion comprises:
(a) displaying the respective first image to a user and receiving an input from the user indicating whether the first region meets the first criterion;
(b) calculating a signal indicating a defect severity in the respective first image, and comparing the calculated signal with a predetermined threshold, a result of the comparing indicating whether the first region meets the first criterion; or
(c) providing the respective first image as input to a trained classifier and receiving a corresponding output from the trained classifier indicating whether the first region meets the first criterion.

7. The computer-implemented method of any one of claims 1-6, further comprising:
performing the subsequent analytic procedure on the given first region.

8. The computer-implemented method of claim 7, wherein the subsequent analytic procedure comprises deposition of an analyte species over a time interval by a deposition beam having a beam axis, and the performing further comprises:
selecting the beam axis and the time interval such that the given first region lies in a predetermined zone of a deposition pattern; and
activating the deposition beam to deposit the analyte species onto the substrate.

9. The computer-implemented method of any one of claims 7-8, wherein the subsequent analytic procedure comprises deposition of an analyte species and the method further comprises, subsequent to the performing:
directing the beam to the given first region;
acquiring a third image of the given first region; and
determining whether the third image meets the first criterion;
determining whether the third image shows presence of a number of analyte particles in a range from one to a predetermined limit; or
outputting a fourth image derived from the third image to be used for characterizing the analyte species.

10. The computer-implemented method of any one of claims 1-9, wherein the substrate is graphene.

11. An apparatus, comprising:
a controller having memory coupled thereto and configured to perform the method of any one of claims 1-10;
a beam source configured to direct the beam to a selected portion of the substrate; and
a detector configured to image the selected portion illuminated by the beam.

12. The apparatus of claim 11, wherein the beam source is an electron source and the beam is an unfocused electron beam having energy between 50 eV and 600 eV.

13. The apparatus of any one of claims 11-12, wherein the detector comprises a pixelated electron detector.
